# EUROPEAN PATENT APPLICATION

(11) **EP 1 588 724 A2**
(43) Date of publication of application: **26.10.2005**
(21) Application number: 05252492.3
(22) Date of filing: 21.04.2005
(51) Int. Cl.: A61L 27/46, A61L 27/56

(54) **Composite material comprising fibrous organic material and fibrous calcium phosphate**

(30) Priority: 22.04.2004 JP 2004127253
(71) Applicant: NGK SPARK PLUG CO., LTD, Mizuho-ku Nagoya-shi Aichi 454 (JP)
(72) Inventor: Mizutani, Yoiohiro, c/o NGK Spark Plug Co. Ltd., Nagoya, Aichi (JP); Nogami, Masayuki, Seto, Aichi (JP); Kasuga, Toshihiro, Q-3 Grand-maison Otai, Nishikasugai, Aichi (JP)
(74) Representative: Nicholls, Michael John

(57) **Abstract**

An organic-inorganic composite porous material containing fibrous calcium phosphate and a fibrous organic material, wherein the fibrous organic material is entangled with at least a portion of the fibrous calcium phosphate; a method for producing a fibrous organic material, which includes preparing a liquid mixture by mixing, with a solution containing a water-soluble polymer and a water-soluble polymer-coagulating agent, a solution prepared by dissolving an organic material in a solvent, and removing the solvent from the liquid mixture while stirring the liquid mixture; and a method for producing an organic-inorganic composite porous material, which includes mixing a fibrous organic material with fibrous calcium phosphate, and subjecting the resultant mixture to adhesion.

## Description

The present invention relates to an organic-inorganic composite porous material, to a method for producing a fibrous organic material, and to a method for producing an organic-inorganic composite porous material. More particularly, the present invention relates to an organic-inorganic composite porous material which can be widely employed as a biomaterial, and in particular, a prosthetic material for tooth or bone, a filler material for a column or the like, or an environmental material adapted for adsorbing contaminants; to a method for producing a fibrous organic material; and to a method for producing an organic-inorganic composite porous material.

Conventionally developed materials for restoring bone or tooth include sintered hydroxyapatite, water-cured calcium phosphate, or polylactic acid (which is a biodegradable resin). These materials have already been put into practice. In recent years, as a next-generation biomaterial, a demand has arisen for a material which has a pore structure that facilitates invasion of living tissues (e.g., cells) therein, which exhibits excellent biocompatibility, and which is gradually absorbed in a living organism. Such a material is also envisaged as a prosthetic material employed for regenerative medicine in which the material is used in combination with cells. Also, a material having a continuous pore structure which enables smooth permeation of a liquid or gas to be isolated, and containing calcium phosphate of high adsorbability is useful as a filler material for a column or the like, or an environmental material used for the purpose of, for example, removing contaminants.

Examples of such a material include various porous materials, such as a calcium phosphate compound exhibiting high bioactivity, a biodegradable resin which is degraded and absorbed in a living organism (e.g., polylactic acid), and a composite material thereof, and these porous materials have conventionally been developed.

One such porous material is a calcium phosphate porous material obtained through a process employing, as a pore-forming agent, a thermally degradable resin or the like. This calcium phosphate porous material contains continuous spherical pores. Therefore, the porous material is disadvantageous in that the diameter of a path between adjacent pores is small, and thus the diameter of the pores must be increased to a size greater than necessary, in order to increase the path diameter.

Meanwhile, as a porous material exhibiting good continuity between pores (hereinafter also referred to as "pore continuity"), an organic-inorganic composite porous material produced by molding a fibrous biodegradable resin containing calcium phosphate has been proposed (Patent Document 1).

### [Patent Document 1]

### Japanese Patent Application Laid-Open (kokai) No. 2003-159321

In order to produce the porous material described in Patent Document 1, a calcium phosphate compound, which is added for imparting bioactivity or a similar property to the porous material, must be mixed with a biodegradable resin in advance. Therefore, the calcium phosphate compound is less exposed on the surface of the porous material, and thus the porous material fails to be sufficiently provided with properties (e.g., bioactivity) of the calcium phosphate; i.e., the effects of the calcium phosphate are not commensurate with the addition amount thereof. Specifically, the majority of the calcium phosphate, which imparts bioactivity to the porous material, is present in the interior of the resin; i.e., the degree of exposure of the calcium phosphate to the outside is very low with respect to the addition amount thereof, which is not desirable.

Meanwhile, a calcium phosphate porous material containing apatite whiskers has conventionally been proposed (Literature Reference 1).

Apatite whiskers have different crystal planes, each plane adsorbing a specific substance. Therefore, a material containing apatite whiskers having a controlled crystal morphology is very useful as a biomaterial or an adsorbent. For example, when a biomaterial containing apatite whiskers, whose specific crystal plane is said to promote adsorption, proliferation, etc., of living tissues (e.g., cells), is implanted in a living organism, the biomaterial facilitates proliferation of cells, etc. Therefore, such an apatite-whisker-containing material is envisaged as a material for promoting restoration of a lost portion of a living organism, or a material for regenerative medicine (e.g., a bioimplant material in which cells, etc., have been cultured in advance). In addition, apatite whiskers whose crystal planes are regulated to adsorb a specific substance can be employed as an environmental material (e.g., an adsorbent of high selectivity).

### [Literature Reference 1]

### "Kagaku Kogyo," September issue, 1993, pp. 710-717

However, the porous material described in the above literature reference, which is produced by sintering apatite whiskers, is disadvantageous in that the apatite whiskers are thermally decomposed during sintering thereof, and thus a limitation is imposed on the composition of the apatite whiskers constituting the porous material. For example, when the Ca/P mole ratio of the apatite whiskers is lower than 1.67, which is the stoichiometric ratio, the apatite whiskers may be thermally decomposed, possibly resulting in generation of a calcium phosphate product other than the apatite.

As described in Patent Document 2, a porous material formed merely of a fibrous biodegradable resin has also been developed. This biodegradable porous material, which exhibits high pore continuity, is useful as a biomaterial at a site which does not require provision of a bioactive substance (e.g., calcium phosphate). However, production of the porous material requires a special fiber production apparatus, since the fibrous biodegradable resin is produced by means of a spraying method. In general, production of resin fiber requires a special apparatus such as a wet-spinning apparatus. The method for producing the calcium-phosphate-containing fibrous biodegradable resin described in Patent Document 1 also requires such a special apparatus. Particularly, the production method described in Patent Document 1, in which calcium phosphate is dispersed in advance in a resin dissolved in a solvent, and the resultant dispersion is formed into fiber filaments by use of a spinning apparatus (e.g., a spraying apparatus), followed by adhesion of the filaments to thereby produce a porous material, is disadvantageous. This is because the subject production method clogs of the spraying apparatus with calcium phosphate particles contained in the resin, and forms short fiber filaments. In general, such a conventional fibrous biodegradable resin produced by use of a spinning apparatus or a similar apparatus, which resin is in the form of long fiber, is suitable for use in a material such as a sheet-like non-woven fabric, but is not suitable for producing a block-like material. Particularly, a fibrous biodegradable resin produced by the spraying method is not suitable for producing a block-like material; i.e., a limitation is imposed on the shape of a material to which the fibrous resin can be applied.

### [Patent Document 2]

### Japanese Patent Application Laid-Open (kokai) No. 2002-315819

In order to solve the aforementioned problems of the prior art, the present invention contemplates provision of an organic-inorganic composite porous material having a continuous pore structure and containing fibrous calcium phosphate which is dispersed therein so as to be generally exposed on the surfaces of pores and on the surface of the porous material; a method for producing a fibrous organic material, which is simpler than a conventional production method and which does not require a special apparatus; and a method for producing the organic-inorganic composite porous material.

The present invention provides means for solving the aforementioned problems, which are summarized as follows.

In a first aspect (1), the invention provides an organic-inorganic composite porous material containing fibrous calcium phosphate and a fibrous organic material, wherein at least a portion of the fibrous organic material is entangled with at least a portion of the fibrous calcium phosphate.

In a preferred embodiment (2), the invention provides an organic-inorganic composite porous material according to (1) above, wherein at portions of entanglement, the fibrous organic material and the fibrous calcium phosphate are joined at least partially.

In another preferred embodiment (3), the invention provides an organic-inorganic composite porous material according to (1) or (2) above, which has a porosity of at least 30%.

In another preferred embodiment (4), the invention provides an organic-inorganic composite porous material according to any one of (1) through (3) above, wherein the fibrous organic material is a biodegradable resin.

In another preferred embodiment (5), the invention provides an organic-inorganic composite porous material according to (4) above, wherein the biodegradable resin contains at least one polymer selected from the group consisting of polylactic acid, polyglycolic acid and polycaprolactone, and/or a copolymer containing a repeating unit derived from at least one monomer selected from the group consisting of lactic acid, glycolic acid and caprolactone.

In another preferred embodiment (6), the invention provides an organic-inorganic composite porous material according to any one of (1) through (5) above, wherein the fibrous calcium phosphate comprises apatite whiskers.

In a second aspect (7), the invention provides a method for producing a fibrous organic material, which comprises a step of preparing a liquid mixture by mixing, with a solution containing a water-soluble polymer and a water-soluble polymer-coagulating agent, a solution prepared by dissolving an organic material in a solvent; and a step of removing the solvent from the liquid mixture while stirring the liquid mixture.

In a preferred embodiment (8), the invention provides a method for producing a fibrous organic material according to (7) above, wherein the water-soluble polymer comprises polyvinyl alcohol.

In a preferred embodiment (9), the invention provides a method for producing a fibrous organic material according to (7) or (8) above, wherein the organic material comprises a biodegradable resin.

In a preferred embodiment (10), the invention provides a method for producing a fibrous organic material according to (9) above, wherein the biodegradable resin contains at least one polymer selected from the group consisting of polylactic acid, polyglycolic acid and polycaprolactone, and/or a copolymer containing a repeating unit derived from at least one monomer selected from the group consisting of lactic acid, glycolic acid and caprolactone.

In a preferred embodiment (11), the invention provides a method for producing a fibrous organic material according to any one of (7) through (10) above, wherein the water-soluble polymer-coagulating agent contains a condensed phosphoric acid salt and/or a sulfuric acid salt.

In a preferred embodiment (12), the invention provides a method for producing a fibrous organic material according to (11) above, wherein the condensed phosphoric acid salt contains at least one species selected from the group consisting of sodium pyrophosphate, sodium tripolyphosphate, and sodium hexametaphosphate.

In a preferred embodiment (13), the invention provides a method for producing a fibrous organic material according to any one of (7) through (12) above, wherein the solvent is selected from the group consisting of methylene chloride, chloroform, dichloroethane and ethyl acetate.

In a third aspect (14), the invention provides a method for producing an organic-inorganic composite porous material, which comprises mixing a fibrous organic material with fibrous calcium phosphate, and at least partially joining the fibrous organic material to the fibrous calcium phosphate through adhesion.

In a preferred embodiment (15), the invention provides a method for producing an organic-inorganic composite porous material according to (14) above, wherein the adhesion is attained through melting of the fibrous organic material.

In a preferred embodiment (16), the invention provides a method for producing an organic-inorganic composite porous material according to (14) or (15) above, wherein the fibrous calcium phosphate comprises apatite whiskers.

In a preferred embodiment (17), the invention provides a method for producing an organic-inorganic composite porous material according to any one of (14) through (16) above, wherein the fibrous organic material comprises a biodegradable resin.

In a preferred embodiment (18), the invention provides a method for producing an organic-inorganic composite porous material according to (17) above, wherein the biodegradable resin contains at least one polymer selected from the group consisting of polylactic acid, polyglycolic acid and polycaprolactone, and/or a copolymer containing a repeating unit derived from at least one monomer selected from the group consisting of lactic acid, glycolic acid and caprolactone.

In a preferred embodiment (19), the invention provides a method for producing an organic-inorganic composite porous material according to any one of (14) through (18) above, wherein the fibrous organic material comprises a fibrous organic material produced by a production method as described in any one of (7) through (13) above.

In a preferred embodiment (20), the invention provides a method for producing an organic-inorganic composite porous material according to any one of (16) through (19) above, wherein the apatite whiskers are formed by a method including a step of preparing a dispersion by dispersing, in a solvent containing at least one of water and a hydrophilic organic solvent, a calcium phosphate gel compound which generates orthophosphoric acid and protons at 80 to 250°C, and a step of heating the resultant dispersion in a sealed container to 80 to 250°C, in which the pH of the dispersion is adjusted to 9 or lower before said heating, and from 3.9 to 9 after said heating.

In a preferred embodiment (21), the invention provides a method for producing an organic-inorganic composite porous material according to any one of (14) through (20) above, wherein soluble particles are added to a mixture of the fibrous organic material and the fibrous calcium phosphate.

In a preferred embodiment (22), the invention provides a method for producing an organic-inorganic composite porous material according to (21) above, wherein the soluble particles are removed by use of a solvent after the fibrous organic material is at least partially joined to the fibrous calcium phosphate through adhesion.

In a preferred embodiment (23), the invention provides a method for producing an organic-inorganic composite porous material according to (21) or (22) above, wherein the soluble particles are formed of a water-soluble polysaccharide and/or a water-soluble inorganic salt.

### Effects of the Invention:

The organic-inorganic composite porous material of the present invention contains fibrous calcium phosphate and a fibrous organic material, in which the fibrous organic material is entangled with at least a portion of the fibrous calcium phosphate, and at least a portion of the entanglement is in a fiber-joining state. Therefore, almost the entirety of the fibrous calcium phosphate contained in the organic-inorganic composite porous material is exposed on the inner surfaces of pores and on the surface of the composite porous material; i.e., there is a high degree of exposure of the fibrous calcium phosphate contained in the organic-inorganic composite porous material. In addition, the organic-inorganic composite porous material has a high porosity, contains pores having a diameter falling within a range of 1 to 1,000 µm, and a highly continuous pore structure. The organic-inorganic composite porous material, which contains the highly exposed fibrous calcium phosphate exhibiting excellent bioactivity and adsorbability and exhibits good pore continuity, can be employed, for example, as a medical material or an environmental material.

In the case where the fibrous organic material is a biodegradable resin, when the organic-inorganic composite porous material is implanted in a lost portion of a living organism, the biodegradable resin is gradually decomposed, and most of the composite porous material is replaced by living tissue, whereby the defect is restored. Therefore, the organic-inorganic composite porous material provided by the present invention, which exhibits good biocompatibility, is suitable for use as a medical material. Furthermore, the organic-inorganic composite porous material, which is also employed as an industrial material (e.g., environmental material), can be treated by biodegradation after use thereof; i.e., the composite porous material is environmentally friendly.

In the case where the biodegradable resin contains at least one polymer selected from the group consisting of polylactic acid, polyglycolic acid and polycaprolactone, and/or a copolymer containing a repeating unit derived from at least one monomer selected from the group consisting of lactic acid, glycolic acid and caprolactone, when the type of the polymer or the copolymer is appropriately selected, organic-inorganic composite porous materials exhibiting various characteristics and properties (ranging from a hard porous material to a spongy porous material) can be provided. The biodegradable resin may be employed in combination with a biodegradable resin other than the aforementioned polymer and copolymer.

According to the present invention, since the organic-inorganic composite porous material contains the fibrous calcium phosphate, continuity between pores formed by the fibrous organic material is not impeded. Since the fibrous calcium phosphate assumes the form of filaments, filaments of the fibrous calcium phosphate are well entangled with one another, or the fibrous calcium phosphate is well entangled with the fibrous organic material. Therefore, even when the amount of the fibrous organic material is small, a porous structure can be formed. In addition, the amount of the fibrous calcium phosphate can be increased.

According to the present invention, an organic-inorganic composite porous material can be provided containing apatite whiskers as the aforementioned fibrous calcium phosphate. The calcium ion site, phosphate ion site, and hydroxyl group site of the apatite whiskers contained in the organic-inorganic composite porous material may be substituted by a variety of ion species, and the characteristics of the resultant apatite whiskers are changed in accordance with the amount or type of the thus-substituted ion species. The type of apatite whiskers contained in the organic-inorganic composite porous material may be selected in accordance with the intended use of the porous material. For example, carbonate apatite, which is obtained through substitution of the phosphate ion site or the hydroxyl group site by a carbonate ion, exhibits a dissolution rate higher than that of hydroxyapatite in a living organism; and fluorapatite, which is obtained through substitution of the hydroxyl group site by a fluoride ion, is more chemically stable than hydroxyapatite. Therefore, through appropriate selection of the type of apatite whiskers to be employed, an organic-inorganic composite porous material can be provided whose dissolution rate is regulated to a level suitable for the site of a living organism to which the porous material is to be applied. Meanwhile, by controlling of the morphology of apatite whiskers, the resultant organic-inorganic composite porous material can be used as an environmental material exhibiting selective adsorbability, or a biomaterial exhibiting characteristics advantageous for cell adhesion or cell growth.

A characteristic feature of the fibrous organic material production method of the present invention resides in that the method includes a step of preparing a liquid mixture by mixing, with an aqueous solution containing a water-soluble polymer and a water-soluble polymer-coagulating agent, a solution prepared by dissolving an organic material in a solvent; and a step of removing the solvent from the liquid mixture while stirring the liquid mixture. Therefore, the production method can readily produce a fibrous organic material without using a special apparatus, unlike the case of a conventional technique such as spraying or wet-spinning. In a preferred mode of the fibrous organic material production method of the present invention, a fibrous organic material is produced as follows. An aqueous solution containing a water-soluble polymer and a water-soluble polymer-coagulating agent is mixed with a solution prepared by dissolving an organic material in a solvent (hereinafter the resultant solution may also be referred to as a "solvent solution"), to thereby form a mixture in which the solvent solution and the aqueous solution are separated from each other. Subsequently, the mixture is subjected to stirring, to thereby obtain a dispersion in which spherical droplets of the solvent solution are dispersed in the aqueous solution. Gradually, the spherical droplets of the solvent solution are dispersed so as to assume a fibrous form by means of the action of the water-soluble polymer and the water-soluble polymer-coagulating agent. Thereafter, the solvent is gradually dissolved in the aqueous solution, and then removed through evaporation, whereby the organic material dissolved in the solvent is precipitated while maintaining its fibrous form, thereby producing a fibrous organic material dispersed in the aqueous solution. The thus-produced fibrous organic material is removed from the aqueous solution by separating the organic material from the aqueous solution through filtration, centrifugation or a similar technique, and the thus-separated organic material is dried. Thus, the production method of the present invention can readily produce a fibrous organic material without using a special apparatus. Therefore, the production method can be employed for producing a raw material of a porous material, as well as a raw material of a typical non-woven fabric. The production method of the present invention can produce short fibers having an average length of some mm or less (in particular, some µm to some mm). The thus-produced short fibers can be employed in materials of various shapes (including a block-like material and a sheet-like material). The short fiber produced through the production method of the present invention can be uniformly mixed with another material, and thus is excellent as a raw material for producing a composite material.

No particular limitations are imposed on the water-soluble polymer employed in the fibrous organic material production method, so long as the polymer coagulates at the interface between the solvent solution and the aqueous solution by means of the water-soluble polymer-coagulating agent which coexists with the polymer in the aqueous solution. From an economical viewpoint, the water-soluble polymer is preferably polyvinyl alcohol. Polyvinyl alcohol is suitable for use as an additive, since it is inexpensive, and, even when remaining in the resultant fibrous organic material, it exhibits relatively high safety to a living organism.

In the fibrous organic material production method, when the organic material is a biodegradable organic material, a fibrous organic material can be produced which is suitable for forming an organic-inorganic composite porous material exhibiting good absorbability in a living organism. For reasons similar to those described for the organic-inorganic composite porous material of the present invention, the biodegradable organic material is preferably at least one polymer selected from the group consisting of polylactic acid, polyglycolic acid and polycaprolactone, and/or a copolymer containing a repeating unit derived from at least one monomer selected from the group consisting of lactic acid, glycolic acid and caprolactone.

In the fibrous organic material production method, the water-soluble polymer-coagulating agent preferably contains a condensed phosphoric acid salt and/or a sulfuric acid salt. The water-soluble polymer-coagulating agent acts as an additive for coagulating the water-soluble polymer added to the aqueous solution. Specifically, when the solvent solution containing the organic material is added to the aqueous solution containing the water-soluble polymer and the water-soluble polymer-coagulating agent, and then the resultant mixture is stirred, by means of the action of the polymer-coagulating agent, membranes of the water-soluble polymer are formed on the surfaces of droplets of the solvent solution which are dispersed in the mixture. When the water-soluble polymer membranes are formed, the form of the stirred solvent solution droplets is changed into a fibrous form by means of the viscosity of the films, and the organic material dissolved in the solvent solution is precipitated in the aqueous solution while maintaining its fibrous form. Therefore, the water-soluble polymer-coagulating agent is an essential additive in the fibrous organic material production method. No particular limitations are imposed on the water-soluble polymer-coagulating agent, so long as it can coagulate the water-soluble polymer. Particularly when the water-soluble polymer is polyvinyl alcohol, the water-soluble polymer-coagulating agent is preferably a condensed phosphoric acid salt and/or a sulfuric acid salt, which exhibits high coagulation activity. More preferably, the water-soluble polymer-coagulating agent is at least one condensed phosphoric acid salt selected from the group consisting of sodium pyrophosphate, sodium tripolyphosphate and sodium hexametaphosphate.

In the fibrous organic material production method, when the solvent is selected from the group consisting of methylene chloride, chloroform, dichloroethane and ethyl acetate, which have low solubility to water and an evaporation temperature lower than that of water, the organic material can be precipitated in a fibrous form in the aqueous solution while being well dispersed therein.

A characteristic feature of the organic-inorganic composite porous material of the present invention, which contains a fibrous organic material and fibrous calcium phosphate, resides in that the fibrous organic material is entangled with at least a portion of the fibrous calcium phosphate, and a portion of the entanglement is in a fiber-joining state. The organic-inorganic composite porous material may be produced by any production method without particular limitation, so long as the method can produce an organic-inorganic composite porous material. Preferably, the organic-inorganic composite porous material is produced by means of the production method of the present invention.

According to the organic-inorganic composite porous material production method of the present invention, the aforementioned organic-inorganic composite porous material can be readily produced by mixing a fibrous organic material with fibrous calcium phosphate, and joining the fibrous organic material to at least a portion of the fibrous calcium phosphate through an adhesion technique.

Examples of the adhesion technique include a technique in which the fibrous organic material and the fibrous calcium phosphate, which are entangled with each other, are immersed in a solvent capable of dissolving the fibrous organic material, to thereby dissolve a portion of the surface of the fibrous organic material, and filaments of the fibrous organic material having dissolved surfaces are bonded to filaments of the fibrous calcium phosphate at points at which the fibrous organic material filaments are in contact with the calcium phosphate filaments; and a technique in which the fibrous organic material is bonded to the fibrous calcium phosphate by melting a portion of the surface of the fibrous organic material through heating. Of these techniques, the adhesion technique employing heating is preferred. The adhesion technique in which the fibrous organic material is bonded to the fibrous calcium phosphate by melting a portion of the surface of the fibrous organic material through heating (hereinafter this technique may also be referred to as a "thermal adhesion technique"), which can be performed through appropriate selection of heating means, is a simple and preferable technique.

The organic-inorganic composite porous material production method of the present invention, which employs fibrous calcium phosphate, can be used to prepare a mixture in which fibrous calcium phosphate and a fibrous organic material are entangled with each other, whereby an organic-inorganic composite porous material of high pore continuity can be more readily produced. In addition, the number of points at which filaments of the fibrous calcium phosphate are in contact with filaments of the fibrous organic material is increased. Therefore the fibrous calcium phosphate is readily borne in the organic-inorganic composite porous material, and the amount of the fibrous calcium phosphate contained therein can be increased.

When apatite whiskers, whose characteristics can be changed through substitution of the ion sites of the whiskers by various ion species, are selected as the fibrous calcium phosphate, through appropriate selection of the type or amount of ion species for substitution, an organic-inorganic composite porous material can be produced whose properties in a living organism (e.g., absorption rate) are controlled. In the production method of the present invention, thermal treatment is performed to an extent such that filaments of the fibrous organic material are joined to one another through adhesion, or the fibrous organic material is joined to the fibrous calcium phosphate through adhesion. Thus, the heating temperature is considerably lower than the sintering temperature employed in a conventional technique. Therefore, even when apatite whiskers are employed as the fibrous calcium phosphate, an organic-inorganic composite porous material can be produced without decomposition of the apatite whiskers.

Particularly, when apatite whiskers are produced through a production method including a step of preparing a dispersion by dispersing, in a solvent containing at least one of water and a hydrophilic organic solvent, a calcium phosphate gel compound which generates orthophosphoric acid and protons at 80 to 250°C, and including a step of heating the resultant dispersion in a sealed container from 80 to 250°C, wherein the pH of the dispersion is adjusted to 9 or lower before heating the dispersion, and from 3.9 to 9 after heating the dispersion, the thus-produced apatite whiskers have a relatively large length; i.e., an average length of 10 µm or more. When such large-length apatite whiskers are employed, advantageously, an organic-inorganic composite porous material containing continuous pores having an average pore diameter of at least 1 µm (particularly 1 µm to 100 µm) can be readily produced.

In the organic-inorganic composite porous material production method, when the fibrous organic material is a biodegradable organic material, an organic-inorganic composite porous material can be produced exhibiting good absorbability in a living organism. Particularly, for reasons similar to those described the organic-inorganic composite porous material of the present invention, the biodegradable organic material is preferably at least one polymer selected from the group consisting of polylactic acid, polyglycolic acid and polycaprolactone, and/or a copolymer containing a repeating unit derived from at least one monomer selected from the group consisting of lactic acid, glycolic acid and caprolactone.

When the fibrous organic material is produced through the aforementioned fibrous organic material production method, the organic-inorganic composite porous material can be readily produced without using a special apparatus. The aforementioned production method may employ a pore-forming agent (e.g., a foaming agent or soluble particles) for producing the organic-inorganic composite porous material of the present invention, which contains fibrous calcium phosphate and a fibrous organic material, characterized in that the fibrous organic material is entangled with at least a portion of the fibrous calcium phosphate, and a portion of the entanglement is in a fiber-joining state. Particularly, a method employing soluble particles serving as a pore-forming agent, which method can readily form pores having a diameter of some tens of µm to some mm, is suitable for control of, for example, pore diameter or porosity. In this method, a mixture of soluble particles, a fibrous organic material, and fibrous calcium phosphate is subjected to thermal treatment or a similar treatment, thereby joining the fibrous organic material to a portion of the fibrous calcium phosphate. Subsequently, the resultant product is immersed in a solvent which can dissolve substantially only the soluble particles, so as to dissolve the particles in the solvent, thereby forming a porous material. The skeleton of the thus-formed porous material containing pores formed by the pore-forming agent has a mesh structure formed of the fibrous calcium phosphate and the fibrous organic material. Therefore, this porous material exhibits excellent continuity between the pores. The diameter of the pores formed by the pore-forming agent, and the diameter of the pores of the mesh structure can be separately controlled in accordance with the intended use of the porous material. The diameter of the pores formed by the pore-forming agent can be controlled by regulating the diameter of the soluble particles. Meanwhile, the diameter of the pores of the mesh structure can be controlled by regulating the form or size of the fibrous calcium phosphate and the fibrous organic material, or regulating the pressure during formation of the porous material. In general, a useful biomaterial contains, in its skeleton, pores having a diameter of some tens of µm or more, which facilitate invasion of living tissues (e.g., cells) therein, as well as pores having a diameter of some µm to some tens of µm, through which a nutrient permeates for promoting growth of living tissues (e.g., cells) in the biomaterial. The present invention can provide a material having such pores. In the composite porous material of the present invention, by virtue of its mesh structure, the fibrous calcium phosphate exhibits its properties efficiently. Therefore, the skeleton of the porous material having a mesh structure exhibits good wettability, and the porous material exhibits excellent nutrient permeability.

Embodiments of the invention will now be described, by way of example only, with reference to the accompanying drawings in which:-
Fig. 1 shows the pore diameter distribution of the organic-inorganic composite porous material of Example 6 as measured using a mercury porosimeter.
Fig. 2 shows a SEM photograph of the organic-inorganic composite porous material produced in Example 5.
Fig. 3 shows a SEM photograph of the organic-inorganic composite porous material produced in Example 6.
Fig. 4 shows a SEM photograph of the organic-inorganic composite porous material produced in Example 7.
Fig. 5 shows a SEM photograph of the organic-inorganic composite porous material produced in Example 8.
Fig. 6 shows a SEM photograph of the organic-inorganic composite porous material produced in Comparative Example 3.
Fig. 7 shows an appearance photograph of an organic-inorganic composite porous material produced in Example 11.

### (1) Organic-inorganic composite porous material

The organic-inorganic composite porous material of the present invention contains fibrous calcium phosphate and a fibrous organic material, in which the fibrous organic material is entangled with at least a portion of the fibrous calcium phosphate, and a portion of the entanglement is in a fiber-joining state.

No particular limitations are imposed on the fibrous organic material employed in the present invention, so long as it is an organic material which assumes the form of a fiber and which can be employed, for example, in a biomaterial, a filler material for a column or the like, or an environmental material used for adsorbing contaminants. Examples of the fibrous organic material employed in a biomaterial include a biodegradable resin. When an organic-inorganic composite porous material containing such a biodegradable resin is implanted in a living organism, the resin is absorbed by the living organism, and bone, etc., are formed.

Particularly preferred examples of the biodegradable resin include at least one polymer selected from the group consisting of polylactic acid, polyglycolic acid and polycaprolactone, and/or a copolymer containing a repeating unit derived from at least one monomer selected from the group consisting of lactic acid, glycolic acid and caprolactone. In the present invention, the fibrous organic material may be selected from among the aforementioned polymers and copolymers, or a combination of at least two selected from among the polymers and copolymers.

These biodegradable resins, which have different properties (e.g., biodegradation rate and strength), can be appropriately selected in accordance with the intended use of the composite porous material. Through selection of the type, average molecular weight, etc., of the biodegradable resin to be employed, a variety of organic-inorganic composite porous materials (ranging from a hard organic-inorganic composite porous material to a spongy, soft organic-inorganic composite porous material) can be produced.

The average length of filaments of the fibrous organic material is generally at least 10 µm, particularly preferably 10 to 10,000 µm. The average length of the fibrous organic material filaments can be readily determined by photographing the filaments by use of an electron microscope or an optical microscope. The fibrous organic material may be employed in combination with an organic material having, for example, a spherical shape or an irregular shape. In this case, the amount of the fibrous organic material is 50 mass% or more, preferably 80 mass% or more, on the basis of the total amount of the fibrous organic material and an organic material other than the fibrous organic material. Each of the filaments of the fibrous organic material may have a non-uniform diameter, and such non-uniformity in diameter does not impede the effects of the present invention. In some cases, a filament of the fibrous organic material employed in the present invention has a structure in which one end is thick and round and the other end is thin and sharp. Such a structural form is also called a "fibrous form."

The amount of the fibrous organic material (e.g., the aforementioned biodegradable resin) contained in the organic-inorganic composite porous material is generally 10 to 99 mass%, particularly 20 to 99 mass%, on the basis of the entirety of the composite porous material. The amount of the fibrous organic material is appropriately determined within the above range. When the amount of the fibrous organic material is less than the above-described lower limit, the strength of the organic-inorganic composite porous material may be lowered, leading to poor handling properties.

The fibrous calcium phosphate employed in the present invention may be one or more selected from hydroxyapatite, carbonate apatite, fluorapatite, calcium hydrogenphosphate, calcium hydrogenphosphate hydrate, α-tricalcium phosphate, β-tricalcium phosphate, tetracalcium phosphate, calcium primary phosphate and calcium primary phosphate hydrate, which calcium phosphate compounds exhibit different dissolution properties. When, for example, the organic-inorganic composite porous material is employed as a biomaterial, the fibrous calcium phosphate to be employed is selected from among the aforementioned compounds for controlling the absorption/substitution rate of the material.

The fibrous calcium phosphate generally assumes the form of a fiber. The fibrous calcium phosphate is preferably in the form of apatite whiskers.

The calcium ion site, phosphate ion site, and hydroxyl group site of the apatite whiskers may be substituted by a variety of ion species, and the characteristics of the apatite whiskers are changed in accordance with the amount or type of the thus-substituted ion species. Particularly, carbonate apatite, which is obtained through substitution of the phosphate ion site and/or the hydroxyl group site by a carbonate ion, exhibits a dissolution rate higher than that of hydroxyapatite in a living organism, whereas fluorapatite, which is obtained through substitution of the hydroxyl group site by a fluoride ion, is more chemically stable than hydroxyapatite. Therefore, through appropriate selection of the type of apatite whiskers to be employed, an organic-inorganic composite porous material can be provided whose dissolution rate is regulated to a level suitable for the site of a living organism to which the porous material is to be applied. The crystal planes of apatite whiskers exhibit different adsorption properties. Therefore, when the morphology of apatite whiskers to be employed is controlled, the degree of exposure of a specific crystal plane is selectively increased, whereby the characteristics of the resultant organic-inorganic composite porous material can be controlled. Thus, the organic-inorganic composite porous material can be used as an environmental material exhibiting selective adsorbability, or a biomaterial exhibiting characteristics advantageous for cell adhesion or cell proliferation.

No particular limitations are imposed on the length of the fibrous calcium phosphate (e.g., apatite whiskers). In order to cause the organic-inorganic composite porous material to have a highly continuous pore structure, the average major axis length of the fibrous calcium phosphate is preferably 10 µm or more, particularly preferably 10 to 1,000 µm. No particular limitations are imposed on the average aspect ratio of the fibrous calcium phosphate, but the aspect ratio is preferably 1 to 1,000, particularly preferably 5 to 500. The average major axis length and average aspect ratio of the fibrous calcium phosphate can be readily determined through observation thereof under an electron microscope or an optical microscope.

Preferably, the fibrous calcium phosphate is produced by means of, for example, a method in which calcium phosphate is mixed with a binder, etc., and the resultant mixture is subjected to extrusion molding, followed by sintering of the molded product; or the hydrothermal method. However, the production method for the fibrous calcium phosphate is not limited to these methods. The fibrous calcium phosphate employed in the present invention may be tricalcium phosphate fiber obtained through thermal treatment of apatite whiskers produced by means of the hydrothermal method.

Apatite whiskers (i.e., a preferred example of the fibrous calcium phosphate) can be produced by a method including a step of preparing a dispersion by dispersing, in a solvent containing at least one of water and a hydrophilic organic solvent, a calcium phosphate gel compound which generates orthophosphoric acid and protons at 80 to 250°C, and a step of heating the resultant dispersion in a sealed container to 80 to 250°C, in which the pH of the dispersion is adjusted to 9 or lower before heating the dispersion, and from 3.9 to 9 after heating the dispersion. This method employs a calcium phosphate gel compound which generates orthophosphoric acid and protons at 80 to 250°C, since apatite whiskers having an average major axis length of at least 10 µm (in particular, 10 µm to 100 µm) can be readily produced in a relatively efficient manner. Examples of the hydrophilic organic solvent include C1-C3 lower alcohols such as methanol and ethanol; ethers such as dimethyl ether, diethyl ether, and methyl ethyl ether; and water-soluble ketones such as acetone. In the heating step, the dispersion is heated to a temperature falling within the above range, in order to decompose the calcium phosphate gel compound, thereby generating orthophosphoric acid and protons. The pH of the dispersion is adjusted to 9 or lower before heating, since, when the pH is higher than 9, apatite whiskers having an average major axis length of at least 10 µm (in particular, 10 µm to 100 µm) fail to be produced. The pH of the dispersion is adjusted so as to fall within the above range after heating, since, when the pH of the dispersion is lower than 3.9, a large amount of calcium hydrogenphosphate (i.e., by-product) is generated, whereas when the pH of the dispersion is higher than 9, apatite whiskers having an average major axis length of at least 10 µm (in particular, 10 µm to 100 µm) fail to be produced.

The calcium phosphate gel compound to be employed is preferably a condensed calcium phosphate gel, which hydrolyzes at 80 to 250°C to generate orthophosphoric acid and protons. Particularly, the condensed calcium phosphate gel to be employed is preferably calcium tripolyphosphate or calcium pyrophosphate. The calcium phosphate gel may contain an excess amount of calcium ion, condensed phosphate ion, etc., for regulation of the ratio by mole between calcium and phosphorus.

The amount of the fibrous calcium phosphate (e.g., apatite whiskers) contained in the organic-inorganic composite porous material is generally 1 to 90 mass%, preferably 1 to 80 mass%, on the basis of the entirety of the composite porous material. No particular limitations are imposed on the amount of the fibrous calcium phosphate, so long as the amount falls within in the above range. The organic-inorganic composite porous material of the present invention has a continuous pore structure, and thus almost the entirety of the surface of the fibrous calcium phosphate is exposed to the outside. Therefore, even when the amount of the fibrous calcium phosphate is small, the composite porous material can be effectively provided with bioactivity and adsorbability.

As described above, the organic-inorganic composite porous material of the present invention contains continuous pores. The porosity of the organic-inorganic composite porous material, which contains continuous pores, is 30% or more, preferably 50 to 95%, particularly preferably 60 to 90%. The diameter of pores of the organic-inorganic composite porous material generally falls within a range of 1 to 1,000 µm. The porosity of the organic-inorganic composite porous material can be calculated from the outside dimensions and weight of the porous material, and the pore diameter can be measured by use of, for example, a mercury porosimeter.

When the organic-inorganic composite porous material of the present invention is employed as a biomaterial, preferably, pores of the porous material having a diameter of 10 to 1,000 µm account for 50% (on a volume basis) or more of all the pores of the porous material, and the porosity of the porous material is 50% or more. As described above, the pore diameter can be measured by use of, for example, a mercury porosimeter.

SEM observation of a broken-out section of the organic-inorganic composite porous material of the present invention (in particular, a composite porous material incorporating apatite whiskers) shows that the fibrous organic material is entangled with the fibrous calcium phosphate, and a portion of the entanglement is in a fiber-joining state. Specifically, the fibrous organic material is entangled with at least a portion of the calcium phosphate, and a portion of the entanglement is in a fiber-joining state, thereby forming a porous structure. Therefore, the composite porous material has a continuous pore structure, and the fibrous calcium phosphate is exposed on the inner surfaces of pores and on the surface of the composite porous material. Specifically, most of the fibrous calcium phosphate is exposed on the inner surfaces of pores and on the surface of the composite porous material. Therefore, the composite porous material exhibits excellent bioaffinity. Furthermore, the composite porous material exhibits wettability to water, since the fibrous calcium phosphate has hydrophilicity. In addition, the composite porous material facilitates invasion of living cells or living tissue therein; i.e., the composite porous material exhibits biocompatibility, since it has a continuous pore structure. Therefore, the composite porous material of the present invention is very useful as a biomaterial. Meanwhile, the composite porous material, which contains the fibrous calcium phosphate exhibiting high adsorbability, is useful as a material which utilizes adsorbability of the fibrous calcium phosphate. Also, the composite porous material is useful as an environmental material, since the porous material contains continuous pores having good permeability of a liquid or gas containing a substance to be adsorbed.

That is, the organic-inorganic composite porous material, which has the above-described unique structure, is suitable for use as a biomaterial, a filler material, or an environmental material.

### (2) Production method for fibrous organic material

The fibrous organic material can be produced by means of the fibrous organic material production method of the present invention. Specifically, the fibrous organic material can be produced through the following procedure: a solvent solution prepared by dissolving an organic material in a solvent is mixed with an aqueous solution containing a water-soluble polymer and a water-soluble polymer-coagulating agent, thereby preparing a liquid mixture, and subsequently the liquid mixture is stirred, followed by removal of the solvent from the liquid mixture. The production method of the present invention can readily produce the fibrous organic material without using a special apparatus, unlike the case of a conventionally known production method; for example, a production method employing spraying, wet-spinning, or a similar technique.

The fibrous organic material production method of the present invention, which is a simple and conventionally unknown method, can be employed for producing a raw material for a porous material, as well as a sheet-like raw material for a non-woven fabric.

No particular limitations are imposed on the organic material, so long as it can be employed as a biomaterial (e.g., an implant material for tooth or bone), a filler material for a column or the like, or an environmental material used for the purpose of adsorbing contaminants. In particular, a biodegradable organic material is preferably employed as a biomaterial.

Examples of the biodegradable organic material include at least one polymer selected from the group consisting of polylactic acid, polyglycolic acid and polycaprolactone, and a copolymer containing, as a repeating unit, at least one monomer selected from the group consisting of lactic acid, glycolic acid and caprolactone. These organic materials are preferred for the reasons described above.

Preferred examples of the solvent for preparing the solvent solution include a solvent which is slightly dissolved in water and which has an evaporation temperature lower than that of water. Examples thereof include at least one solvent selected from the group consisting of methylene chloride, chloroform, dichloroethane and ethyl acetate.

The concentration of the organic material in the solvent solution is generally the saturation concentration or a lower concentration.

The aqueous solution must contain a water-soluble polymer. When a water-soluble polymer is contained in the aqueous solution, a fibrous organic material can be precipitated by the effects of the water-soluble polymer and water-soluble polymer-coagulating agent contained in the aqueous solution.

No particular limitations are imposed on the water-soluble polymer, so long as it coagulates at the interface between the solvent solution and the aqueous solution by means of the action of the water-soluble polymer-coagulating agent present in the aqueous solution. From an economical viewpoint, the water-soluble polymer is particularly preferably polyvinyl alcohol. Polyvinyl alcohol is suitable for use as an additive, since it is inexpensive, and, even when remaining in the resultant fibrous organic material, polyvinyl alcohol exhibits relatively high safety to a living organism. No particular limitations are imposed on the amount of the water-soluble polymer contained in the aqueous solution, since the optimum amount of the water-soluble polymer to be employed varies depending on the type or molecular weight thereof. However, the amount of the water-soluble polymer is preferably 0.001 mass% to 10 mass%.

The water-soluble polymer-coagulating agent preferably contains a condensed phosphoric acid salt and/or a sulfuric acid salt. The water-soluble polymer-coagulating agent is particularly preferably condensed sodium phosphate or sodium sulfate. The effect of a water-soluble polymer-coagulating agent may vary in accordance with the pH of the aqueous solution. Therefore, an acid or an alkali, serving as a pH adjusting agent, may be added to the aqueous solution containing the water-soluble polymer-coagulating agent. For example, when sodium sulfate, which exhibits its effect under alkali conditions, is employed as the water-soluble polymer-coagulating agent, preferably, an alkali (e.g., sodium hydroxide) is added to the aqueous solution containing sodium sulfate.

Examples of the condensed phosphoric acid salt include sodium pyrophosphate, sodium tripolyphosphate and sodium hexametaphosphate. The condensed phosphoric acid salt contained in the aqueous solution may be one or two or more selected from among the aforementioned condensed phosphoric acid salts.

The concentration of the condensed phosphoric acid salt in the aqueous solution is generally 0.001 to 1 M. When the concentration is too low, the condensed phosphoric acid salt fails to exhibit the effect of forming the organic material into a fibrous material, whereas when the concentration is excessively high, a precipitate of the condensed phosphoric acid salt may be generated, which is undesirable.

The mixing ratio by volume of the solvent solution prepared by dissolving an organic material in a solvent to the aqueous solution containing a water-soluble polymer and a water-soluble polymer-coagulating agent is preferably 1:1 to 1:500. When the mixing ratio by volume of the solvent solution to the aqueous solution, which varies depending on the type of the solvent or organic material, is regulated so as to fall within the above range, the organic material is readily formed into a good fibrous material through precipitation in the aqueous solution.

In the method of the present invention, generally, the solvent solution is mixed with the aqueous solution at ambient temperature. If desired, mixing of the solvent solution with the aqueous solution may be performed under heating or cooling conditions.

The solvent solution may be mixed with the aqueous solution under stirring. Alternatively, stirring may be performed after mixing the solvent solution with the aqueous solution. Preferably, the mixture of the solvent solution and the aqueous solution is stirred sufficiently. When the mixture is stirred, the solvent solution is dispersed in the aqueous solution, and, in the resultant dispersion, merely the solvent is gradually solubilized at the interface between the solvent solution and the aqueous solution. As a result, the solvent, which has an evaporation temperature lower than that of water, is evaporated from the dispersion, whereby the concentration of the organic material contained in the solvent solution is increased. Finally, the organic material is formed into a fibrous material through precipitation in the aqueous solution. Stirring of the mixture is performed at ambient or reduced pressure under heating conditions or at ambient temperature. Stirring of the mixture can be performed by use of a stirring apparatus such as a stirrer. The length of the resultant fibrous organic material can be controlled by regulating the stirring speed. The higher the stirring speed, the shorter the length of the fibrous organic material, whereas the lower the stirring speed, the longer the length of the fibrous organic material.

After the mixture is stirred sufficiently, and then the solvent is evaporated, the fibrous organic material dispersed in the aqueous solution is separated using a technique such as filtration or centrifugation, followed by drying of the thus-separated fibrous organic material by use of, for example, a dryer. When a biodegradable resin is employed as the organic material, preferably, the fibrous organic material is dried by means of vacuum drying, since the biodegradable resin may be decomposed through heating.

The fibrous organic material may be purified by means of a generally employed chemical washing or purification technique. For example, the fibrous organic material is completely washed with pure water, and then the wet fibrous organic material is dried, to thereby remove the water-soluble polymer, etc., deposited on the surface of the fibrous organic material.

### (3) Production method for organic-inorganic composite porous material

A characteristic feature of the organic-inorganic composite porous material production method of the present invention resides in that a fibrous organic material is mixed with fibrous calcium phosphate, to thereby entangle the fibrous organic material with at least a portion of the fibrous calcium phosphate. Furthermore, a mode of the entanglement is joining. The production method can readily produce the organic-inorganic composite porous material of the present invention. The organic-inorganic composite porous material has a pore structure of high continuity, in which the fibrous calcium phosphate is exposed on the inner surfaces of pores and on the surface of the porous material.

In the organic-inorganic composite porous material production method of the present invention, particularly preferably, the fibrous organic material is joined to the fibrous calcium phosphate by means of an adhesion technique in which a portion of the fibrous organic material and a portion of the fibrous calcium phosphate are melted by heating under pressurized conditions or at ambient pressure, and the thus-melted portions are bonded to each other. Alternatively, for example, a portion of the fibrous organic material and a portion of the fibrous calcium phosphate may be bonded to each other by use of an organic material serving as an adhesive. In the case of this adhesion technique, filaments of the fibrous organic material are joined to one another.

In addition to the aforementioned thermal adhesion technique, a technique may be employed in which a mixture of the fibrous organic material and the fibrous calcium phosphate is brought into contact with a solvent capable of dissolving the fibrous organic material such that the surface of the fibrous organic material is dissolved, followed by drying, to thereby join the fibrous organic material to the fibrous calcium phosphate. Similar to the case of the aforementioned thermal adhesion technique, in the case of this technique, filaments of the fibrous organic material are joined to one another. This technique, in which a portion of the surface of the fibrous organic material is dissolved in a solvent, to thereby join the fibrous organic material to the fibrous calcium phosphate and between filaments of the fibrous organic material, may be classified as a "solvent-employing adhesion technique." In the solvent-employing adhesion technique, no particular limitations are imposed on the amount of the solvent to be employed, so long as the surface of the fibrous organic material is dissolved in the solvent. The type and amount of the solvent to be employed may be appropriately selected in accordance with the type of the fibrous organic material.

The organic-inorganic composite porous material production method of the present invention, in which a fibrous organic material is mixed with fibrous calcium phosphate, and the resultant mixture is subjected to molding under heating, can produce an organic-inorganic composite porous material. The thus-produced organic-inorganic composite porous material (i.e., the organic-inorganic composite porous material of the present invention) has a highly continuous pore structure, contains pores having a diameter falling within a range of 10 to 1,000 µm, and has high porosity. Furthermore, almost the entirety of the fibrous calcium phosphate contained in the organic-inorganic composite porous material is exposed on the inner surfaces of the pores and on the surface of the composite porous material. Namely, a large portion of the fibrous calcium phosphate contained in the organic-inorganic composite porous material is exposed.

The mixing ratio of the fibrous organic material to the fibrous calcium phosphate is appropriately determined in accordance with the intended use of the organic-inorganic composite porous material, such that the amounts of the fibrous organic material and fibrous calcium phosphate contained in the composite porous material fall within the above-described corresponding ranges.

The fibrous organic material-fibrous calcium phosphate mixture is preferably prepared by mixing fibrous calcium phosphate with a fibrous organic material produced by the fibrous organic material production method of the present invention, although the mixture may be prepared by mixing an isolated fibrous organic material with isolated fibrous calcium phosphate.

No particular limitations are imposed on the method for mixing the fibrous organic material and the fibrous calcium phosphate. For example, the mixture can be prepared by the following procedure: the fibrous organic material and the fibrous calcium phosphate are dispersed in pure water, the resultant dispersion is subjected to filtration, and the thus-separated solid product is dried. In this case, dispersion of these materials can be performed by means of, for example, a treatment employing a stirrer, or an ultrasonic treatment. When the fibrous calcium phosphate content is high, the resultant organic-inorganic composite porous material exhibits improved bioaffinity, but the strength of the composite porous material is lowered. This is because the number of points at which filaments of the fibrous organic material are in contact with filaments of the fibrous calcium phosphate, or the number of points at which filaments of the fibrous organic material are in contact with one another, is reduced. In contrast, when the fibrous calcium phosphate content is low, the resultant organic-inorganic composite porous material exhibits enhanced strength, but the bioaffinity of the composite porous material is reduced. Therefore, the compositional ratio between the fibrous calcium phosphate and the fibrous organic material must be determined in accordance with the site of a living body to which the resultant organic-inorganic composite porous material is to be applied.

A pore-forming agent (e.g., a foaming agent or soluble particles) may be employed during production of the organic-inorganic composite porous material, to thereby control the porosity and the pore diameter. From the viewpoint of convenience in operation, soluble particles are particularly preferably employed. Soluble particles (i.e., particles which can be dissolved in water, etc.) are added to the fibrous organic material-fibrous calcium phosphate mixture, and the resultant mixture is subjected to molding under heating. Thereafter, the molded product is immersed in a solvent (e.g., water) or subjected to a similar treatment, and merely the soluble particles are dissolved in the solvent, to thereby form pores. The skeleton of the thus-produced organic-inorganic composite porous material containing pores formed by the pore-forming agent has a mesh structure formed of the fibrous calcium phosphate and the fibrous organic material. Therefore, the composite porous material exhibits excellent continuity between the pores. The diameter of the pores formed by the pore-forming agent, and the diameter of the pores of the mesh structure can be separately controlled in accordance with the intended use of the composite porous material. The diameter of the pores formed by the pore-forming agent can be controlled by regulating the diameter of the soluble particles.

Meanwhile, the diameter of the pores of the mesh structure can be controlled by regulating the form or size of the fibrous calcium phosphate and the fibrous organic material, or regulating the pressure during formation of the porous material.

Examples of the solvent employed in the present invention include solvents which do not substantially dissolve the fibrous organic material and the fibrous calcium phosphate constituting the organic-inorganic composite porous material and which can dissolve soluble particles. Examples of such solvents include water, ethanol and methanol. Of these, water is preferred. Preferred examples of the soluble particles include particles which can be dissolved in the aforementioned solvent (in particular, water). The soluble particles are preferably formed of, for example, a water-soluble polysaccharide and/or a water-soluble inorganic salt. Examples of the water-soluble polysaccharide include sucrose, dextran, and a dextran sulfate salt. Examples of the water-soluble inorganic salt include alkali metal halides such as sodium chloride and potassium chloride; and alkaline earth metal halides such as calcium chloride.

When the average particle diameter of the soluble particles is excessively small, the soluble particles fail to serve as a pore-forming agent, whereas when the average particle diameter is excessively large, it is difficult to uniformly distribute pores in the porous material. Therefore, the average particle diameter is preferably regulated so as to fall within a range of 10 µm to 2 mm. No particular limitations are imposed on the amount of the soluble particles to be added, but the amount must be regulated to an optimum level in accordance with the intended porosity of the organic-inorganic composite porous material whose skeleton has a mesh structure. Preferably, the amount of the soluble particles is regulated such that the porosity of the organic-inorganic composite porous material becomes 30% or more.

In the present invention, after mixing the fibrous organic material with the fibrous calcium phosphate, the resultant mixture is subjected to molding under heating.

The heating temperature, which varies depending on the type of the fibrous organic material, is generally regulated to a temperature falling within a range of the softening temperature of the fibrous organic material to the decomposition temperature thereof. Specifically, the heating temperature is preferably regulated to 50 to 300°C, particularly preferably 100 to 250°C.

When the heating temperature is regulated so as to fall within the above range, portions at which filaments of the fibrous organic material are in contact with one another, or portions at which filaments of the fibrous organic material are in contact with filaments of the fibrous calcium phosphate are in a fiber-joining state through melting of the fibrous organic material, whereby an organic-inorganic composite porous material having a continuous pore structure can be readily produced. Thus, the production method of the present invention can produce an organic-inorganic composite porous material at a temperature lower than the firing temperature of a ceramic material (e.g., fibrous calcium phosphate). Therefore, the production method can readily avoid decomposition of the added fibrous calcium phosphate, and is advantageous in terms of production cost.

When the heating temperature is low, portions at which filaments of the fibrous organic material are in contact with one another, or portions at which filaments of the fibrous organic material are in contact with filaments of the fibrous calcium phosphate may fail to be joined to one another through melting of the fibrous organic material. Consequently, the strength of the organic-inorganic composite porous material may be considerably lowered, leading to poor handling thereof. In contrast, when the heating temperature is excessively high, the entirety of the fibrous organic material may be melted, whereby pores may fail to be formed. Therefore, the heating temperature is appropriately determined in accordance with the type, molecular weight, etc., of the fibrous organic material. Heating of the fibrous organic material-fibrous calcium phosphate mixture may be performed simultaneous with molding of the mixture, or may be performed after molding of the mixture. The mixture may be pressurized during molding.

Molding of the fibrous organic material-fibrous calcium phosphate mixture is performed through the following procedure: the mixture is charged into a mold selected in accordance with the intended use of the organic-inorganic composite porous material, the mixture is heated to the aforementioned heating temperature, and, if desired, the resultant product is pressurized.

The fibrous calcium phosphate employed in the present invention may comprise at least one member selected from hydroxyapatite, carbonate apatite, fluorapatite, calcium hydrogenphosphate, calcium hydrogenphosphate hydrate, α-tricalcium phosphate, β-tricalcium phosphate, tetracalcium phosphate, calcium primary phosphate and calcium primary phosphate hydrate, which fibrous calcium phosphate compounds exhibit different dissolution properties. When, for example, the organic-inorganic composite porous material is employed as a biomaterial, the fibrous calcium phosphate to be employed is selected from among the aforementioned compounds for controlling the absorption/substitution rate of the material.

The fibrous calcium phosphate generally assumes the form of a fiber. The fibrous calcium phosphate is preferably in the form of apatite whiskers. The fibrous calcium phosphate, which assumes a fiber form, is well entangled with the fibrous organic material, whereby an organic-inorganic composite porous material containing continuous pores can be readily produced. Because of an increase in the number of points at which filaments of the fibrous calcium phosphate are in contact with filaments of the fibrous organic material, as well as entanglement between filaments of the fibrous calcium phosphate, entanglement between filaments of the fibrous organic material, and entanglement of the fibrous calcium phosphate with the fibrous organic material, the fibrous calcium phosphate is readily fixed in the organic-inorganic composite porous material. Furthermore, the amount of the fibrous calcium phosphate contained therein can be increased. When the amount of the fibrous calcium phosphate contained in the organic-inorganic composite porous material is increased, the resultant composite porous material can have excellent pore structure without impeding pore continuity.

The organic-inorganic composite porous material of the present invention, or the organic-inorganic composite porous material produced through the production method of the present invention may be employed as a carrier for a drug delivery system on which a drug (e.g., bone morphogenetic protein) is to be carried, or may be employed as a carrier for regenerative medicine, which is employed after cells, etc., are carried or cultured thereon. The organic-inorganic composite porous material of the present invention may be employed in a form suitable for a site to which the porous material is to be applied (e.g., a granular form or a block form).

### Examples

The present invention will next be described in more detail with reference to Examples and Comparative Examples, but the present invention is not limited to the

### Examples.

### Example 1: Production method for fibrous organic material

Polylactic acid having an average molecular weight of 160,000 (1 g) was dissolved in methylene chloride (15 mL), to thereby prepare a solution. The solution was added to an aqueous solution (500 mL) containing sodium tripolyphosphate (concentration: 0.1 M) and polyvinyl alcohol having an average molecular weight of 22,000 (concentration: 0.01 mass%). While the resultant liquid mixture was stirred for 24 hours, the methylene chloride dispersed in the mixture was removed through evaporation, to thereby precipitate, in the resultant aqueous mixture, the polylactic acid which had been dissolved in the methylene chloride. Thereafter, the resultant aqueous mixture was subjected to filtration. The resultant product was washed with pure water, and then dried in a reduced-pressure desiccator. The thus-produced fibrous polylactic acid product was found to have a length of about 100 µm to 2 mm.

### Example 2: Production method for fibrous organic material

Polylactic acid having an average molecular weight of 160,000 (1 g) was dissolved in methylene chloride (15 mL), to thereby prepare a solution. The solution was added to an aqueous solution (500 mL) containing sodium tripolyphosphate (concentration: 0.1 M) and polyvinyl alcohol having an average molecular weight of 1,500 (concentration: 0.01 mass%). While the resultant liquid mixture was stirred for 24 hours, the methylene chloride dispersed in the mixture was removed through evaporation, to thereby precipitate, in the resultant aqueous mixture, the polylactic acid which had been dissolved in the methylene chloride. Thereafter, the resultant aqueous mixture was subjected to filtration. The thus-separated solid product was washed with pure water, and the thus-washed solid product was dried in a reduced-pressure desiccator. The thus-produced fibrous polylactic acid product was found to have an average length of about 1 mm.

### Example 3: Production method for fibrous organic material

Polylactic acid having an average molecular weight of 160,000 (1 g) was dissolved in methylene chloride (15 mL), to thereby prepare a solution. The solution was added to an aqueous solution (500 mL) containing sodium sulfate (concentration: 0.2 M), polyvinyl alcohol having an average molecular weight of 22,000 (concentration: 0.01 mass%), and sodium hydroxide serving as a pH adjusting agent. The pH of the aqueous solution was found to be 9. While the resultant liquid mixture was stirred for 24 hours, the methylene chloride dispersed in the mixture was removed through evaporation, to thereby precipitate, in the resultant aqueous mixture, the polylactic acid which had been dissolved in the methylene chloride. Thereafter, the resultant aqueous mixture was subjected to filtration. The thus-separated solid product was washed with pure water, and the thus-washed solid product was dried in a reduced-pressure desiccator. The thus-produced fibrous polylactic acid product was found to have an average length of about 0.5 mm.

### Comparative Example 1

Polylactic acid having an average molecular weight of 160,000 (1 g) was dissolved in methylene chloride (15 mL), to thereby prepare a solution. The solution was added to an aqueous solution (500 mL) containing polyvinyl alcohol having an average molecular weight of 22,000 (concentration: 0.01 mass%). While the resultant liquid mixture was stirred for 24 hours, the methylene chloride dispersed in the mixture was removed through evaporation, to thereby precipitate, in the resultant aqueous mixture, the polylactic acid which had been dissolved in the methylene chloride. Thereafter, the resultant aqueous mixture was subjected to filtration. The thus-separated solid product was washed with pure water, and the thus-washed solid product was dried in a reduced-pressure desiccator. The thus-produced polylactic acid product was found to be a mixture of spherical polylactic acid particles and irregularly shaped polylactic acid particles. That is, a fibrous polylactic acid product was not produced, because a water-soluble polymer-coagulating agent was not employed.

### Comparative Example 2

Polylactic acid having an average molecular weight of 160,000 (1 g) was dissolved in methylene chloride (15 mL), to thereby prepare a solution. The solution was added to an aqueous solution (500 mL) containing sodium tripolyphosphate so that the polylactic acid concentration was adjusted to 0.1 M. While the resultant liquid mixture was stirred for 24 hours, the methylene chloride dispersed in the mixture was removed through evaporation, to thereby precipitate, in the resultant aqueous mixture, the polylactic acid which had been dissolved in the methylene chloride. Thereafter, the resultant aqueous mixture was subjected to filtration. The thus-separated solid product was washed with pure water, and the thus-washed solid product was dried in a reduced-pressure desiccator. The thus-produced polylactic acid product was found to have an irregular shape. That is, a fibrous polylactic acid product was not produced, because a water-soluble polymer was not employed. Examples 4 through 8

In Examples 4 through 8, the fibrous polylactic acid product synthesized in Example 1 was employed.

Hydroxyapatite whiskers employed in Examples 4 through 8 were prepared as follows. Specifically, nitric acid was added to a gel containing sodium tripolyphosphate (0.025 M), calcium nitrate (0.125 M), and propanol (30 vol.%), to thereby prepare a dispersion having a pH of 2.4. The dispersion was thermally treated in a sealed container at 140°C for 24 hours. Subsequently, the thus-treated dispersion was subjected to filtration, and the thus-separated solid product was dried. The pH of the resultant filtrate (i.e., pH of the dispersion after thermal treatment) was measured, and found to be 4.1. The thus-produced hydroxyapatite whiskers were found to have an average length of about 60 µm. The fibrous polylactic acid product (a predetermined weight) and the hydroxyapatite whiskers (a predetermined weight) were stirred in ethanol, followed by filtration. The thus-separated solid product was dried under reduced pressure, to thereby yield a mixture of the fibrous organic material and the fibrous calcium phosphate. The mixture was charged into a mold having a diameter of 6 mm and a height of 5 mm, and thermally treated at 170°C to 180°C, followed by cooling, to thereby produce an organic-inorganic composite porous material.

### Examples 9 through 11

In Examples 9 through 11, the fibrous polylactic acid product synthesized in Example 1 was employed. In Examples 9 through 11, sieved sucrose particles (size: 355 to 850 µm) were employed as soluble particles. Hydroxyapatite whiskers employed in Examples 9 through 11 were prepared in a manner similar to that described in Examples 4 through 8. The fibrous polylactic acid product (a predetermined weight), the sucrose particles (a predetermined weight), and the hydroxyapatite whiskers (a predetermined weight) were added to ethanol, to thereby prepare a dispersion. The dispersion was stirred, and then subjected to filtration. The thus-separated solid product was dried under reduced pressure, to thereby yield a mixture of the fibrous organic material, the fibrous calcium phosphate, and the soluble particles. The mixture was charged into a mold having a diameter of 6 mm and a height of 8 mm, a pressure of 100 Mpa was unidirectionally applied to the mixture, and the mixture was thermally treated at 170 to 180°C. Thereafter, the pressure-molded product was removed from the mold, and then immersed in pure water, to thereby dissolve, in the water, the soluble particles contained in the molded product. The resultant columnar product was removed from the pure water, and then dried under reduced pressure, to thereby produce a columnar organic-inorganic composite porous material having a diameter of 6 mm and a height of 8 mm.

Table 1 shows the compositional proportions of hydroxyapatite whiskers and polylactic acid, the porosity of each of the thus-produced organic-inorganic composite porous materials, the amount by volume of pores having a diameter of 10 µm or more as measured by use of a mercury porosimeter, and the water permeation time. The water permeation time was obtained through the following procedure: one droplet of pure water (10 µL) was added to the surface of the organic-inorganic composite porous material by use of a micropipette, and the time required for the water droplet to completely permeate into the organic-inorganic composite porous material was measured.

SEM observation of a broken-out section of each of the organic-inorganic composite porous materials of Examples 4 through 11 showed that filaments of the fibrous polylactic acid were entangled with the hydroxyapatite whiskers, and adhesion occurred at a portion of points at which the filaments and the whiskers were in contact with one another. That is, each of the porous materials of Examples 4 through 11 was found to be the organic-inorganic composite porous material in which the fibrous organic material is entangled with the fibrous calcium phosphate, and a portion of the entanglement is in a fiber-joining state. The SEM observation also showed that fine hydroxyapatite whiskers adhered to the surface of the fibrous polylactic acid.

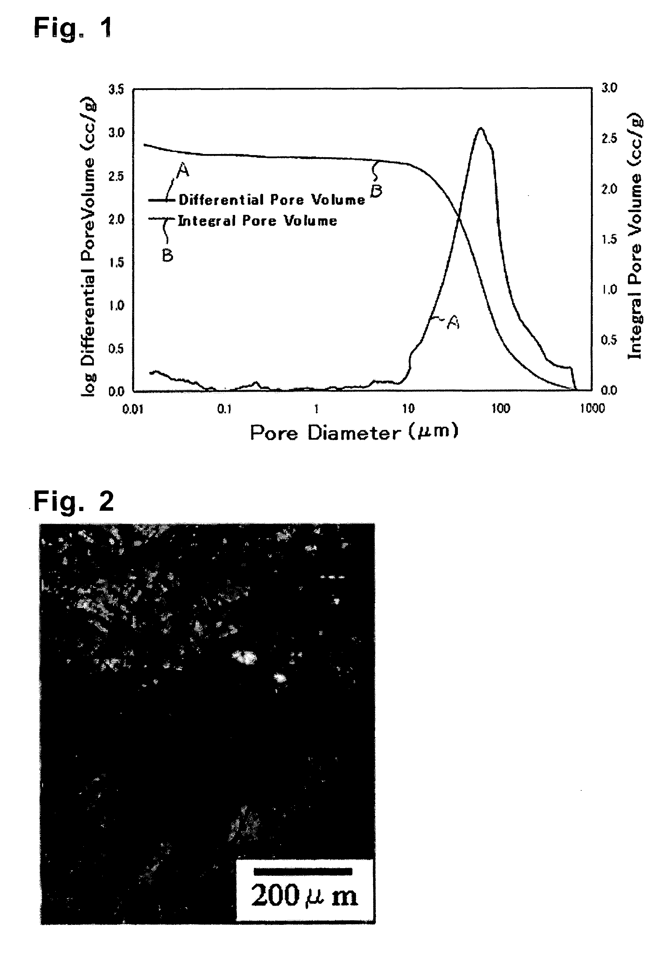

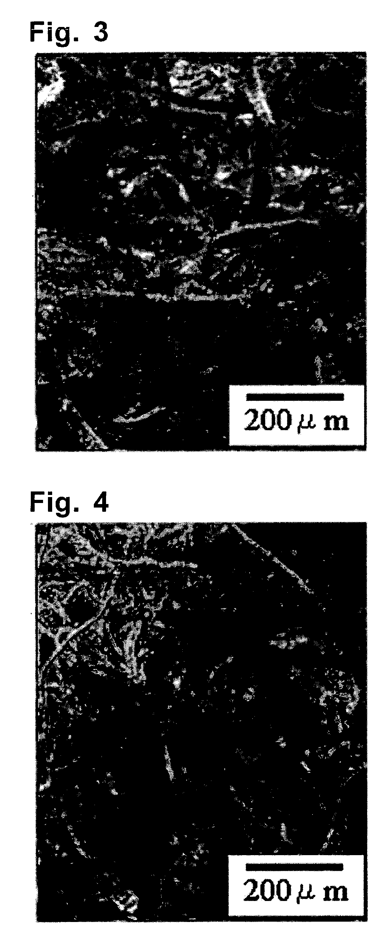

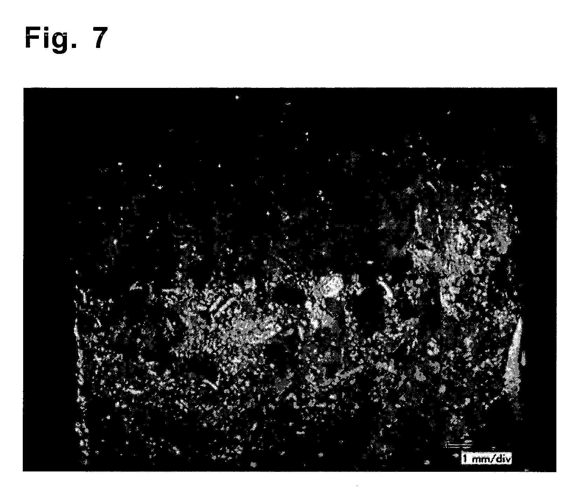

Figs. 2 through 5 show SEM photographs of the organic-inorganic composite porous materials produced in Examples 5 through 8 as representative examples. Fig. 7 shows a photograph of the appearance of the organic-inorganic composite porous material produced in Example 11, which is a representative example of the organic-inorganic composite porous materials produced in Examples 9 through 11. As is clear from Fig. 7, large pores are formed through addition of soluble particles. The surface of the organic-inorganic composite porous material shown in Fig. 7 was subjected to carbon deposition, in order to bring out the contrast between the pores and the surface of the organic-inorganic composite porous material for easy observation of the pores.

### Comparative Example 3

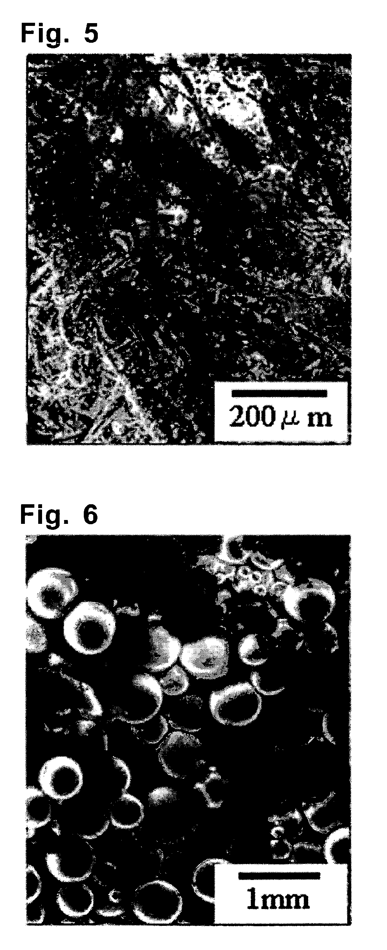

Low-crystallinity apatite employed in Comparative Example 3 was prepared through the following procedure: calcium carbonate and calcium hydrogenphosphate were wet-mixed by use of a ball mill; the resultant mixture was subjected to filtration; the thus-separated solid product was dried; and the thus-dried product was applied to a 150-µm sieve. SEM observation of the low-crystallinity apatite showed that the apatite was in the form of aggregated particles having an average particle size of 8 µm. The low-crystallinity apatite (0.1 g) was added to a solution prepared by dissolving polylactic acid having an average molecular weight of 160,000 (1 g) in methylene chloride (10 mL), to thereby yield a suspension in which the low-crystallinity apatite was dispersed in the polylactic acid solution. The suspension was added to an aqueous solution (500 mL) containing polyvinyl alcohol having an average molecular weight of 22,000 (concentration: 0.02 mass%), followed by stirring for 24 hours, to thereby prepare an aqueous mixture. The aqueous mixture was subjected to filtration, the thus-separated solid product was washed with pure water, and the thus-washed solid product was dried in a reduced-pressure desiccator. Through this procedure, a spherical polylactic acid product containing the low-crystallinity apatite was produced. The spherical polylactic acid product was found to have an average diameter of 600 µm. The spherical polylactic acid product was charged into a disk-shaped mold having a diameter of 7 mm and a height of 5 mm, and the mold was placed in a dryer of 170°C, to thereby produce an organic-inorganic composite porous material containing the apatite in an amount of 10 mass%. Fig. 6 shows an SEM photograph of the organic-inorganic composite porous material produced in Comparative Example 3 as a representative example. The water permeability of the thus-produced organic-inorganic composite porous material was evaluated. In a manner similar to that described above in the Examples, the water permeability was evaluated by adding a droplet of pure water to the surface of the porous material, and measuring the time required for the water droplet to completely permeate into the porous material. As a result, the water droplet did not completely permeate into the organic-inorganic composite porous material even 30 minutes after addition of the droplet. It is considered that this phenomenon is based on the fact that the organic-inorganic composite porous material of Comparative Example 3 is formed of calcium phosphate and spherical polylactic acid, and the amount of the calcium phosphate exposed on the inner surfaces of pores is small.

### Comparative Example 4

In Comparative Example 4, the low-crystallinity apatite employed in Comparative Example 3 and the fibrous polylactic acid product synthesized in Example 1 were employed. The fibrous polylactic acid product (a predetermined weight) and the low-crystallinity apatite (a predetermined weight) were stirred in ethanol, followed by filtration. The thus-separated solid product was dried under reduced pressure, to thereby yield a mixture of the fibrous organic material and the fibrous calcium phosphate. The mixture was charged into a mold having a diameter of 6 mm and a height of 5 mm, and thermally treated at 170°C to 180°C, followed by cooling, to thereby produce an organic-inorganic composite porous material.

A comparison among Examples 1 through 3 and Comparative Examples 1 and 2 reveals that successful production of a fibrous organic material requires both a water-soluble polymer-coagulating agent (e.g., a condensed phosphoric acid salt or sodium sulfate) and a water-soluble polymer (e.g., polyvinyl alcohol), and that a fibrous organic material is readily produced through the procedure described in Examples 1 through 3 without using a special apparatus.

The results of Examples 4 through 8 reveal that when fibrous calcium phosphate is mixed with a fibrous organic material, and the resultant mixture is subjected to molding under heating, the organic-inorganic composite porous material of the present invention is produced, in which the fibrous organic material is entangled with the fibrous calcium phosphate, and at least a portion of the entanglement is in a fiber-joining state. In addition, the fact that the shape of thus-produced porous material reflects the shape (block shape) of the employed mold suggests that porous materials of different shapes can be produced by selecting molds of different shapes.

The results of Examples 9 through 11 reveal that when fibrous calcium phosphate, a fibrous organic material, and soluble particles (e.g., sucrose particles) are mixed together, and the resultant mixture is subjected to molding under heating, followed by immersion of the thus-molded product in pure water and drying of the molded product, an organic-inorganic composite porous material of the present invention is produced, in which the fibrous organic material is entangled with the fibrous calcium phosphate, and at least a portion of the entanglement is in a fiber-joining state.

Each of the organic-inorganic composite porous materials of Examples 4 through 8 contains the fibrous calcium phosphate and the fibrous organic material, wherein the fibrous organic material is joined to at least a portion of the fibrous calcium phosphate (i.e., apatite whiskers). Therefore, the organic-inorganic composite porous material has a highly continuous pore structure, in which pores of the porous material having a diameter of 10 µm or more account for 80% (on a volume basis) or more of all the pores of the porous material. In contrast, in the case of the organic-inorganic composite porous material of Comparative Example 4, which employed aggregated particles of low-crystallinity apatite as calcium phosphate, pores of the porous material having a diameter of 10 µm or more accounted for 50% (on a volume basis) or less of all the pores of the porous material. In the case of the organic-inorganic composite porous material of Example 5, pores of the porous material having a diameter of 10 µm or more accounted for 80% (on a volume basis) or more of all the pores of the porous material, although the amount of the calcium phosphate employed therein was equal to that of the calcium phosphate employed in the porous material of Comparative Example 4. This is because the porous material of Example 5 employed apatite whiskers as the calcium phosphate. This fact suggests that formation of a highly continuous pore structure requires the use of fibrous calcium phosphate. When a composite porous material is produced from aggregated particles of low-crystallinity apatite and fibrous polylactic acid, because the apatite particles are not entangled with the fibrous polylactic acid, the apatite particles tend to fall from the porous material. For example, when the porous material is patted, a portion of the apatite particles falls therefrom. In contrast, when a composite porous material is produced from apatite whiskers and fibrous polylactic acid, because the apatite whiskers are entangled with fibrous polylactic acid, the apatite whiskers tend not to fall from the porous material.

In each of the organic-inorganic composite porous materials of Examples 9 through 11, pores of the porous material having a diameter of 10 µm or more accounted for 60% (on a volume basis) or more of all the pores of the porous material. Observation of the pore structure of the porous material under an electron microscope or an optical microscope showed that the porous material contains large pores having a diameter of 100 µm or more, and the skeleton of the porous material is formed of a mesh structure. The diameter of pores forming the mesh structure was found to be 1 µm to some tens of µm as measured by use of a mercury porosimeter. Therefore, the porous material serves as a scaffold for promoting cell proliferation and tissue growth, and has a structure exhibiting excellent nutrient permeability; i.e., the porous material is useful as a biomaterial.

Each of the organic-inorganic composite porous materials of Examples 4 through 11 had a porosity as high as 60 to 90%. Thus, the results of the Examples show that the organic-inorganic composite porous material of the present invention, which facilitates invasion of living tissue or cells therein and exhibits biocompatibility, is useful as a medical material, and that the porous material, which exhibits good permeability of gas or liquid, is useful as an environmental material or a filler material.

Each of the organic-inorganic composite porous materials of Examples 4 through 11 was found to have excellent water permeability in that a water droplet permeated into the porous material immediately after addition of the water droplet to the surface thereof. These results show that a large amount of the hydrophilic, fibrous calcium phosphate is exposed on the surface of the organic-inorganic composite porous material, and thus the fibrous calcium phosphate exhibits its properties effectively. The water permeation time of the porous material of Example 4 greatly differed from that of the porous material of Comparative Example 3, although the amount of the hydroxyapatite employed in the porous material of Example 4 was equal to that of the hydroxyapatite employed in the porous material of Comparative Example 3. That is, each of the organic-inorganic composite porous materials of the Examples, in which the fibrous organic material is entangled with the fibrous calcium phosphate, effectively utilizes the properties of the fibrous calcium phosphate. Therefore, when the organic-inorganic composite porous material of the present invention is employed as a medical material, the bioactivity of the fibrous calcium phosphate can be utilized effectively, whereas when the porous material is employed as an environmental material or a filler material, the adsorbability of the fibrous calcium phosphate (in particular, apatite) can be utilized; i.e., the porous material is very useful.

**Table 1**

| | Compositional proportions (mass%) | | | | Properties | | |
|---|---|---|---|---|---|---|---|
| | Amount of Polylactic acid | Calcium phosphate | | Sucrose | Porosity (%) | Amount by volume of pores of 10 µm or more (%) | Permeation time (min.) |
| | | Type | Amount | Amount | | | |
| Ex. 4 | 90 | HAp whisker | 10 | - | 80 | 89 | < 1 |
| Ex. 5 | 50 | | 50 | | 79 | 86 | < 1 |
| Ex. 6 | 40 | | 60 | | 84 | 84 | < 1 |
| Ex. 7 | 30 | | 70 | | 82 | 81 | < 1 |
| Ex. 8 | 20 | | 80 | | 84 | 96 | < 1 |
| Ex. 9 | 35 | | 15 | 50 | 62 | 64 | < 1 |
| Ex. 10 | 28 | | 12 | 60 | 72 | 75 | < 1 |
| Ex. 11 | 21 | | 9 | 70 | 78 | 81 | < 1 |
| Comp. Ex. 3 | 90 | Low-crystallinity HAp | 10 | | 60 | 89 | > 30 |
| Comp. Ex. 4 | 50 | | 50 | | 84 | 46 | < 1 |
| * HAp: Hydroxyapatite | | | | | | | |

It should further be apparent to those skilled in the art that various changes in form and detail of the invention as shown and described above may be made. It is intended that such changes being included within the spirit and scope of the claims appended hereto.

This application is based on Japanese Patent Application No. 2004-127253 filed April 22, 2004, incorporated herein by reference in its entirety.

## Claims

1. An organic-inorganic composite porous material comprising fibrous calcium phosphate and a fibrous organic material, wherein at least a portion of the fibrous organic material is entangled at entanglement portions with at least a portion of the fibrous calcium phosphate.

2. An organic-inorganic composite porous material according to claim 1, wherein fibers of the fibrous organic material are at least partially joined to fibers of the fibrous calcium phosphate at the entanglement portions.

3. An organic-inorganic composite porous material according to claim 1 or 2, having a porosity of at least 30%.

4. An organic-inorganic composite porous material according to any one of claims 1 to 3, wherein the fibrous organic material comprises a biodegradable resin.

5. An organic-inorganic composite porous material according to claim 4, wherein the biodegradable resin comprises at least one polymer selected from the group consisting of polylactic acid, polyglycolic acid and polycaprolactone, and/or a copolymer containing at least one repeating unit derived from a monomer selected from the group consisting of lactic acid, glycolic acid and caprolactone.

6. An organic-inorganic composite porous material according to any one of claims 1 to 5, wherein the fibrous calcium phosphate comprises apatite whiskers.

7. A method for producing a fibrous organic material, which comprises preparing a liquid mixture by mixing, with a solution containing a water-soluble polymer and a water-soluble polymer-coagulating agent, a solution prepared by dissolving an organic material in a solvent; and removing the solvent from the liquid mixture while stirring the liquid mixture.

8. A method for producing a fibrous organic material according to claim 7, wherein the water-soluble polymer comprises polyvinyl alcohol.

9. A method for producing a fibrous organic material according to claim 7 or 8, wherein the organic material comprises a biodegradable resin.

10. A method for producing a fibrous organic material according to claim 9, wherein the biodegradable resin comprises at least one polymer selected from the group consisting of polylactic acid, polyglycolic acid and polycaprolactone, and/or a copolymer containing a repeating unit derived from at least one monomer selected from the group consisting of lactic acid, glycolic acid and caprolactone.

11. A method for producing a fibrous organic material according to any one of claims 7 to 10, wherein the water-soluble polymer-coagulating agent contains a condensed phosphoric acid salt and/or a sulfuric acid salt.

12. A method for producing a fibrous organic material according to claim 11, wherein the water-soluble polymer-coagulating agent contains a condensed phosphoric acid salt selected from the group consisting of sodium pyrophosphate, sodium tripolyphosphate and sodium hexametaphosphate.

13. A method for producing a fibrous organic material according to any one of claims 7 to 12, wherein the solvent is selected from the group consisting of methylene chloride, chloroform, dichloroethane and ethyl acetate.

14. A method for producing an organic-inorganic composite porous material, which comprises mixing a fibrous organic material with fibrous calcium phosphate, and at least partially joining through adhesion fibers of the fibrous organic material to fibers of the fibrous calcium phosphate.

15. A method for producing an organic-inorganic composite porous material according to claim 14, wherein said joining comprises melting the fibrous organic material to at least partially join through adhesion fibers of the fibrous organic material to fibers of the fibrous calcium phosphate.

16. A method for producing an organic-inorganic composite porous material according to claim 14 or 15, wherein the fibrous calcium phosphate comprises apatite whiskers.

17. A method for producing an organic-inorganic composite porous material according to any one of claims 14 to 16, wherein the fibrous organic material comprises a biodegradable resin.

18. A method for producing an organic-inorganic composite porous material according to claim 17, wherein the biodegradable resin contains at least one polymer selected from the group consisting of polylactic acid, polyglycolic acid and polycaprolactone, and/or a copolymer containing a repeating unit derived from at least one monomer selected from the group consisting of lactic acid, glycolic acid and caprolactone.

19. A method for producing an organic-inorganic composite porous material according to any one of claims 14 to 18, wherein the fibrous organic material is produced by a method as recited in any one of claims 7 to 13.

20. A method for producing an organic-inorganic composite porous material according to any one of claims 16 to 19, wherein the apatite whiskers are formed by a method which comprises preparing a dispersion by dispersing, in a solvent containing at least one of water and a hydrophilic organic solvent, a calcium phosphate gel compound which generates orthophosphoric acid and protons at 80 to 250°C, and heating the resultant dispersion in a sealed container to 80 to 250°C, in which the pH of the dispersion is adjusted to 9 or lower before said heating, and from 3.9 to 9 after said heating.

21. A method for producing an organic-inorganic composite porous material according to any one of claims 14 to 20, which comprises adding soluble particles to a mixture of the fibrous organic material and the fibrous calcium phosphate.

22. A method for producing an organic-inorganic composite porous material according to claim 21, which comprise removing the soluble particles by use of a solvent after the fibrous organic material and the fibrous calcium phosphate are at least partially joined through adhesion.

23. A method for producing an organic-inorganic composite porous material according to claim 21 or 22, wherein the soluble particles comprise a water-soluble polysaccharide and/or a water-soluble inorganic salt.
